# EUROPEAN PATENT APPLICATION

(11) **EP 4 737 576 A1**
(43) Date of publication of application: **06.05.2026**
(21) Application number: 23946259.1
(22) Date of filing: 27.07.2023
(51) Int. Cl.: C12N 15/85, C12N 15/12, C12N 15/65, C12N 15/90, C12N 15/113, C12N 9/22, A01K 67/027, C12N 5/0781, G01N 33/68

(54) **CONSTRUCTION AND USE OF MOUSE MODEL FOR EFFICIENTLY OBTAINING TUMOR ANTIGEN-SPECIFIC B CELLS**

(71) Applicant: Beijing Institute for Stem Cell and Regenerative Medicine, Beijing 100101 (CN)
(72) Inventor: ZHAO, Tongbiao, Beijing 100101 (CN); ZHU, Yingjie, Beijing 100101 (CN); ZHANG, Lin, Beijing 100101 (CN); CHENG, Chenxi, Beijing 100101 (CN); CAO, Jiani, Beijing 100101 (CN); LI, Xiaoyan, Beijing 100101 (CN)
(74) Representative: Acapo Onsagers AS
(86) International application number: PCT/CN2023/109595
(87) International publication number: WO 2025/020173

(57) **Abstract**

The present invention relates to the field of tumor immunology, and particularly relates to a gene editing-based animal model, for efficiently and rapidly screening for tumor antigen-specific B cells, obtaining antibody information, and providing a new target for clinical personalized treatment.

## Description

### TECHNICAL FIELD

The present disclosure belongs to the field of tumor immunotherapy and relates to a gene editing-based animal model, which is used for rapidly and efficiently sorting tumor antigen-specific B cells; further screening of the sorted B cells enables the acquisition of tumor immunotherapy antibodies or engineered therapeutic cells. In particular, the present disclosure relates to a method for preparing a transgenic C57BL/6 mouse model having a Nur77^{SynNotch}Rosa26^{mCherry} dual-signal sensing system capable of labeling B cells specifically activated by tumor antigens and the use thereof.

### BACKGROUND

Cancer poses a serious threat to human life and health worldwide. In recent years, tumor immunotherapy has achieved significant outcomes in clinical treatment, and was ranked by Science as the first of the top ten scientific breakthroughs in 2013. Chimeric Antigen Receptor T (CAR-T) cell immunotherapy targeting CD19 in particular demonstrated significant remission rates in the clinical treatment of hematological tumors. However, the lack of tumor-specific antigens has severely restricted the further application of tumor immunotherapy in the clinical treatment of solid tumors due to off-target toxic and side effects. Achieving precise recognition of tumor cells has become one of the important strategies for optimizing tumor immunotherapy at present. The rapid development of high-throughput sequencing technology has made it possible to conduct in-depth genomic and transcriptomic analyses of tumor tissues. These analyses facilitate the further characterization and classification of tumors in clinical setting and may also enable the screening of tumor-specific antigens. Nevertheless, firstly, the complexity and heterogeneity of solid tumor tissues render the screening of tumor antigen targets challenging; secondly, comprehensive genomic profiling of solid tumor tissues may not directly yield antibody sequences applicable to corresponding immunotherapies. These limit the clinical advancement of tumor immunotherapy.

The immune system's surveillance function ensures immune homeostasis in the organism. B cells may produce specific antibodies against specific antigen epitopes, and such specific recognition is reflected in the unique variable region sequences of B cell receptors. In recent years, an increasing number of studies have revealed the important role of tertiary lymphoid structures formed with the participation of B cells in anti-tumor processes. Currently, the acquisition of most antigen-specific monoclonal antibodies (mAbs) still relies on conventional hybridoma technology and phage display technology. With the rapid development of single-cell sequencing technology, mAb sequences that recognize specific protein antigens may be rapidly and efficiently obtained by sorting and sequencing B cells that bind to specific modified antigens. However, the method for capturing antigen-specific B cells relies on the *in vitro* expression, purification and modification of known protein antigens, and may not be applied to the screening of B cells that recognize unknown antigens (uAgs) on the surface of tumor cell membranes. Therefore, developing a method for the rapid identification and acquisition of tumor antigen-specific B cells is one of the important strategies to accelerate the clinical application of antibody-mediated tumor immunotherapy.

Nur77 (NR4A1), as an orphan nuclear receptor, may be rapidly upregulated following induction by T/B cell antigen receptor (TCR/BCR) activation signals. Additionally, Nur77 has been reported to serve as a reporter gene for specific TCR or BCR activation signals. The SynNotch receptor is a customizable molecular sensor developed by the Lim laboratory in 2016, which may achieve the programming of cells. Cells may recognize a specific antigen structure via the SynNotch receptor and execute a series of pre-designed responses. Through combinatorial expression applications, cells may be engineered to recognize multiple target antigens and their output responses may be comprehensively programmed. This allows the SynNotch system in the T cells to be activated by the first type of antigen on the surface of tumor cell membranes, which in turn activates the transcription of CAR that recognizes the second type of antigen on the surface of tumor cell membranes. Ultimately, the CAR-T cells may only be activated when both antigens are expressed on tumor cells, thereby enabling precise recognition and killing of tumor cells and reducing damage to normal tissue cells. To capture tumor antigen-specific B cells, we designed a Nur77^{SynNotch}Rosa26^{mCherry} dual-signal sensing system. When the B cell receptor (BCR) recognizes and is activated by the unknown antigen (uAg) on the surface of tumor cell membranes, the orphan nuclear receptor Nur77 is upregulated and drives the localized expression of the SynNotch receptor on the surface of B cell membranes. After the nanobody of the SynNotch receptor outside the membrane recognizes the green fluorescent protein (GFP) tag on the surface of tumor cell membranes, the intracellular artificial transcription factor Gal4-VP64 is cleaved and released by a hydrolase, translocates into the nucleus and initiates the expression of mCherry red fluorescent protein, thus labeling B cells that recognize the uAg-mGFP dual antigens on the surface of tumor cell membranes.

### SUMMARY

The object of the present disclosure is to provide a gene-edited animal model, which is intended to provide a method for efficiently screening B cells specific to unknown tumor antigens on the membrane of specific tumor cells, and aims to solve the technical problem that B cells targeting tumor cell antigens may not be directly and accurately labeled at present.

To solve the above-mentioned technical problems, the present disclosure is achieved by the following technical solution:
1) construction of transgenic vector: the present disclosure relates to a SynNotch receptor expression sequence and a mCherry selection sequence that are inserted at the Nur77 gene locus and the Rosa26 gene locus, respectively. Both the full-length sequences are synthesized by Sangon Biotech (Shanghai) Co., Ltd. The construction of transgenic vector comprises: adding cleavage sites before and after SynNotch receptor expression sequence, and then inserting the SynNotch receptor expression sequence and the cleavage sites between upstream and downstream homologous arms before stop codon of the gene Nur77; and then ligating the gene Nur77, inserted with the SynNotch receptor expression sequence and the cleavage sites, into plasmid pBluescript II SK; adding cleavage sites before and after mCherry selection sequence, inserting the mCherry selection sequence and the cleavage sites between the upstream and downstream homologous arms of the intron of the Rosa26 gene, and then the Rosa26 gene, inserted with the mCherry selection sequence and the cleavage sites, into plasmid pBluescript II SK to obtain a transgenic vector pBluescript II SK-Rosa26mCherry; ligating the sgRNAs of the knock-in sites of the two sequences into the DNA cleavage plasmid pX330-U6-Chimeric_BB-CBh-hSpCas9, respectively. The present disclosure also relates to cell lines that over-express membrane-localized hCD19 protein or GFP protein, comprising constructing plasmids pCDH-mGFP and pCDH-mCD19 by using the lentiviral vector pCDH-CAG as the template: fusing the extracellular region sequence of hCD19 protein or the full-length sequence of GFP protein with the transmembrane domain sequence of human platelet-derived growth factor (PDGF), respectively, to form the mhCD19 and mGFP protein sequences localized on the membrane surface; submitting the protein sequences to Sangon Biotech (Shanghai) Co., Ltd. for codon optimization and synthesis, and then ligating into the lentiviral vector pCDH-CAG via the NotI and SalI cleavage sites, followed by sequencing and expression verification.

The SynNotch receptor expression system has the nucleotide sequence of SEQ ID NO: 1; the mCherry screening system has the nucleotide sequence of SEQ ID NO:2; the upstream homologous arm located before the stop codon of the Nur77 gene has the sequence of SEQ ID NO:3; the downstream homologous arm located before the stop codon of the Nur77 gene has the sequence of SEQ ID NO:4; the upstream homologous arm of the intron of the Rosa26 gene has the sequence of SEQ ID NO:5; and the downstream homologous arm of the intron of the Rosa26 gene has the sequence of SEQ ID NO: 6. The Nur77 sgRNA has the sequence of SEQ ID NO:7; and the Rosa26 sgRNA has the sequence of SEQ ID NO:8. The vector of the membrane-localized GFP protein has the sequence of SEQ ID NO:9; the vector of the membrane-localized hCD19 protein has the sequence of SEQ ID NO:10; the full-length sequence of the Nur77 KI plasmid has the sequence of SEQ ID NO:11; the full-length sequence of the Nur77 sRNA plasmid has the sequence of SEQ ID NO: 12; the full-length sequence of the Rosa26 KI plasmid has the sequence of SEQ ID NO: 13; the full-length sequence of the Rosa26 sRNA plasmid has the sequence of SEQ ID NO:14; the full-length sequence of the vector of the membrane-localized GFP protein has the sequence of SEQ ID NO:15; and the full-length sequence of the vector of the membrane-localized hCD19 protein has the sequence of SEQ ID NO:16.

2) Acquisition of gene-edited mouse: combining the transgenic vector pBluescript II SK-Nur77SynNotch and the Nur77-sgRNA-pX330-hSpCas9 plasmid, and combining the pBluescript II SK-Rosa26mCherry plasmid and the Rosa26-sgRNA-pX330-hSpCas9 plasmid to obtain combinations, and introducing the combinations into fertilized ova of the C57B6L/J mice via microinjection, respectively, implanting the fertilized ova of the C57B6L/J mice into the uteri of pseudopregnant mice to obtain a F0 generation Nur77^{SynNotch}- or Rosa26^{mCherry}-knock-in mice(The mice give birth 19-21 days after the surgery. When the offspring mice are 3 weeks old, toe clipping is carried out, followed by numbering and tail clipping. Genomic DNA is extracted from each mouse. PCR technology is used to detect the integration status of the target genes, thereby obtaining the F0 generation Nur77^{SynNotch} or Rosa26^{mCherry} knock-in mice.); selecting positive mice carrying a single target gene knock-in and crossing with wild-type C57B6L/J mice to obtain F1 generation mice, extracting the genome of the F1 generation mice, and conducting PCR to verify the insertion of a single target gene (either Nur77^{SynNotch} or Rosa26^{mCherry}); intercrossing positive F1 generation mice carrying a single target gene knock-in to produce F2 generation mice; extracting the genome of F2 generation mice, and conducting PCR to verify the simultaneous knock-in of the two target genes Nur77^{SynNotch}Rosa26^{mCherry}; selecting positive F2 generation mice with the co-insertion of both target genes for male-female mating to obtain F3 generation mice. extracting the genome of F3 generation mice, and conducting PCR to verify the co-insertion of the two target genes Nur77^{SynNotch}Rosa26^{mCherry}; further identifying the genotype of the double knock-in target genes by PCR, and selecting homozygous mice; thus, obtaining and preserving homozygous Nur77^{SynNotch}Rosa26^{mCherry} double knock-in mice.

3) Acquisition of tumor antigen-specific B cell: subjecting Nur77^{SynNotch}Rosa26^{mCherry} double knock-in mice immunized with repeated tumor cell inoculations to splenic challenge immunization with mGFP protein-labeled tumor cells. After 48 h, euthanizing the challenge-immunized mice, and preparing the spleen tissues into single-cell suspensions, washing twice with DPBS; resuspending the cell pellets in 1 mL of tissue cell diluent (< 10⁸ cells); obtaining lymphocyte by centrifuging mouse lymphocyte separation solution, following by washing the cells with DPBS, resuspending and counting the cells; enriching B cells using the Mouse Pan B Isolation Kit from Miltenyi; adjust the final concentration of B cells to 1 × 10⁷ cells/mL after the B cell pellets are resuspended. Subsequently, staining the B cells with APC anti-mouse CD19 and FITC anti-mouse IgD flow cytometry direct-labeling antibodies, following by sorting of tumor antigen-specific B cells (CD19+IgD-mCherry+) at the single-cell level.

4) Screening of tumor antigen-specific antibody: obtaining antibodies via single-cell PCR and *in vitro* expression, and identifying the antibody activity by flow cytometry (FCM); digesting A549-mOVA-mCherry and A549-mhCD19 cells, washing the cells once with DPBS, and then counting; Subsequently, collecting a total of 1 × 10⁶ tumor cells at a 1 : 1 ratio of the two cell types, washing the cells once with DPBS, and subjecting to flow cytometric staining using 1 µg of recombinant mAb as the primary antibody and FITC anti-mouse-IgG (1 : 500) as the secondary antibody; finally, analyzing the fluorescence ratios of mCherry and FITC using FCM, thereby evaluating the recognition ability of the recombinant mAb to the protein antigen on tumor cell membranes.

Compared with the prior art, the beneficial effects achieved by the present disclosure mainly include but are not limited to the following aspects:
The present disclosure provides a gene-edited animal model, which may be used to efficiently screen for tumor antigen-specific B cells and rapidly obtain therapeutic antibodies or cell therapy products targeting tumor antigens, overcoming the technical defects of high cost, heavy workload and long time cycle in the existing antibody screening technologies.

The gene-edited animal model provided by the present disclosure may be applicable to a variety of labeled tumor cells, has broader applicability, and may be used to efficiently screen for and obtain B cells that specifically recognize target cell antigens.

The gene-edited animal model provided by the present disclosure may be used to screen for specific B cells that directly recognize and bind to antigens on the surface of target cell membranes (not limited to tumor cells). For all cell membrane protein antigens, the screening of specific B cells may be achieved.

The gene-edited animal model provided by the present disclosure may also achieve the screening and identification of B cells that recognize clinical tumor antigens through humanized modification, and may be used to efficiently screen for tumor antigens that induce immune responses, thereby achieving personalized therapy for clinical tumor treatment.

The gene-edited animal model provided by the present disclosure provides an *in vivo* anti-tumor immune environment, which may better simulate the complex microenvironment of clinical tumors. It is one of the important conditions that are difficult to consider and achieve with *in vitro* screening systems, and achieves unexpected screening effects for tumor antigens.

### BRIEF DESCRIPTION OF THE DRAWINGS

FIG. 1 shows the gene design principle of a gene-edited mouse model. a shows the specific elements of the SynNotch receptor expression sequence and mCherry selection sequence, b shows the design principle of the Nur77^{SynNotch}Rosa26^{mCherry} system, c shows the map of the Nur77 KI plasmid, d shows the map of the Rosa26 KI plasmid, e shows the map of the Nur77 gRNA plasmid, f shows the map of the Rosa26 gRNA plasmid, g shows the map of the PCDH-mGFP plasmid and h shows the map of the PCDH-mCD19 plasmid;
FIG. 2 shows the gene identification of the gene-edited mouse. a shows the design of the identification primer and b shows the result of the mouse gene identification electrophoresis;
FIG. 3 shows the sorting of tumor antigen-specific B cells and the acquisition of antibodies. a shows the strategic method for immunizing mice with tumor cells, b shows the flow cytometric sorting of mCherry positive tumor antigen-specific B cells (CD19+IgD-mCherry+), and c shows the electrophoretic verification results of the BCR heavy chain-light chain sequences obtained by single cell PCR on monoclonal B cells;
FIG. 4 shows the screening of tumor antigen-specific recombinant antibodies. A shows a protocol and strategy for flow analysis of binding and recognition capability of antibodies, and b shows the flow cytometry detection of tumor cell recognition by recombinant antibodies;
FIG. 5 shows the detection of tumor antigen-specific recombinant antibody-dependent cell-mediated cytotoxicity. a shows human-derived tumor cells, and b shows mouse-derived tumor cells.

### DETAILED DESCRIPTION OF THE EMBODIMENTS

To enable those skilled in the art to better understand the technical solutions in the present application, the present disclosure will be described more clearly and completely below in conjunction with specific embodiments of the present application. Obviously, the described embodiments are only some embodiments of the present application, and not all embodiments. All other embodiments obtained by a person of ordinary skill in the art based on the embodiments of the present application without creative efforts shall fall within a protection scope of the present disclosure.

### Example 1

### Screening system design and plasmid construction.

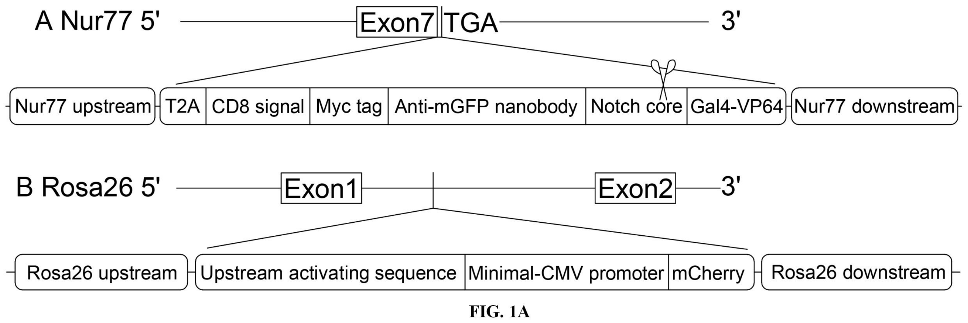

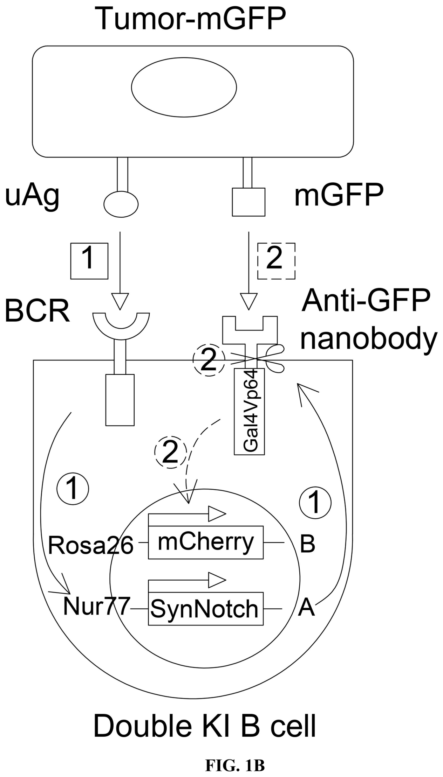

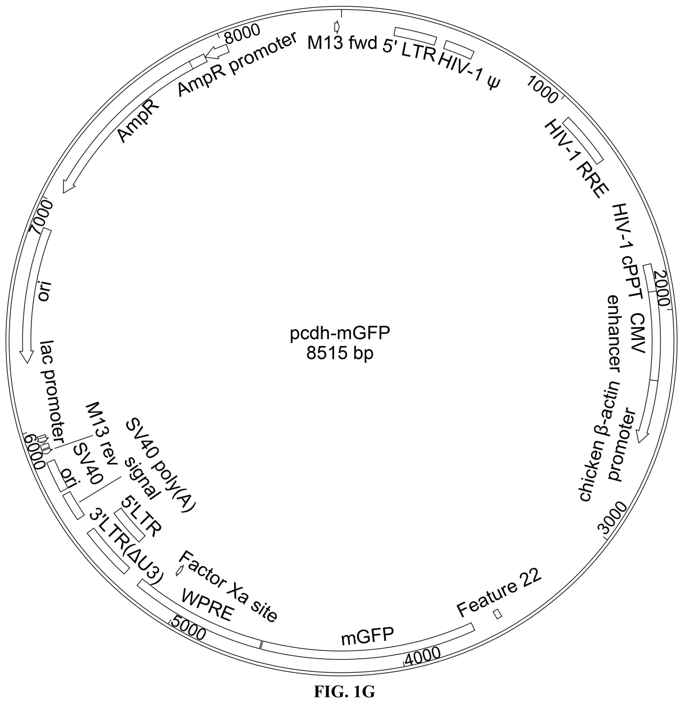

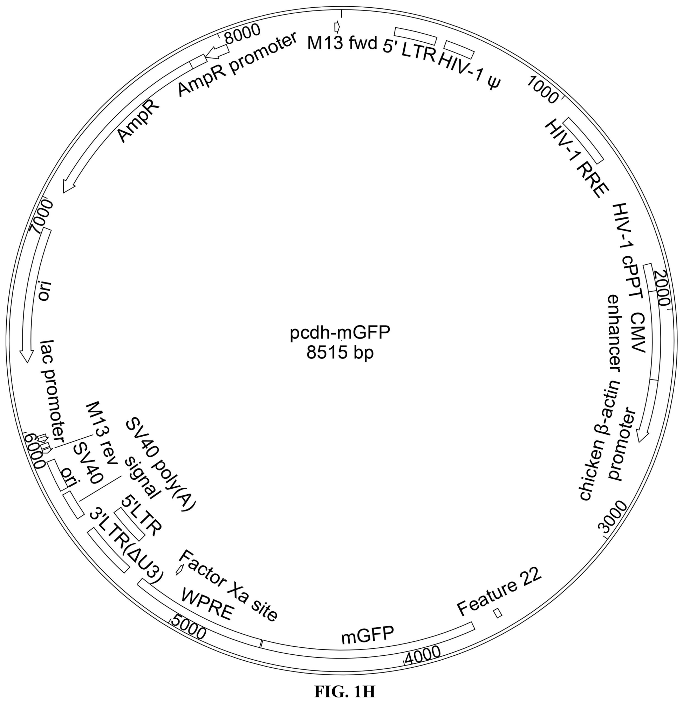

The screening system included a receptor expression system and an activation and screening system. The receptor expression system included a leader sequence, a tag sequence, an extracellular receptor sequence, a transmembrane region, and an intracellular activation reporter; and the activation and screening system included a binding domain, a promoter, and a marker gene (see Engineering Customized Cell Sensing and Response Behaviors Using Synthetic Notch Receptors, Cell, 2016) (FIG. 1a). The Nur77^{SynNotch}Rosa26^{mCherry} dual-antigen sensing system mainly included two-step signal transduction: first, following the activation of BCR upon specific recognition of uAg on the surface of tumor cell membranes, the expression of Nur77 was rapidly upregulated, which drove the localized expression of the SynNotch receptor inserted before the stop codon on the surface of B cell membranes. Second, after the nanobody of the SynNotch receptor outside the membrane specifically bound to the mGFP protein on the surface of tumor cell membranes, the intracellular domain, i.e., artificial transcription factor Gal4-VP64 was enzymatically cleaved from the cell membrane, released, translocated into the nucleus, and drove the expression of red fluorescent protein mCherry (FIG. 1b). In Nur77^{SynNotch}Rosa26^{mCherry} double knock-in mice, relying on the bispecific recognition of uAg-mGFP, B cells reactive to specific tumor cells could be labeled with red fluorescent protein mCherry.

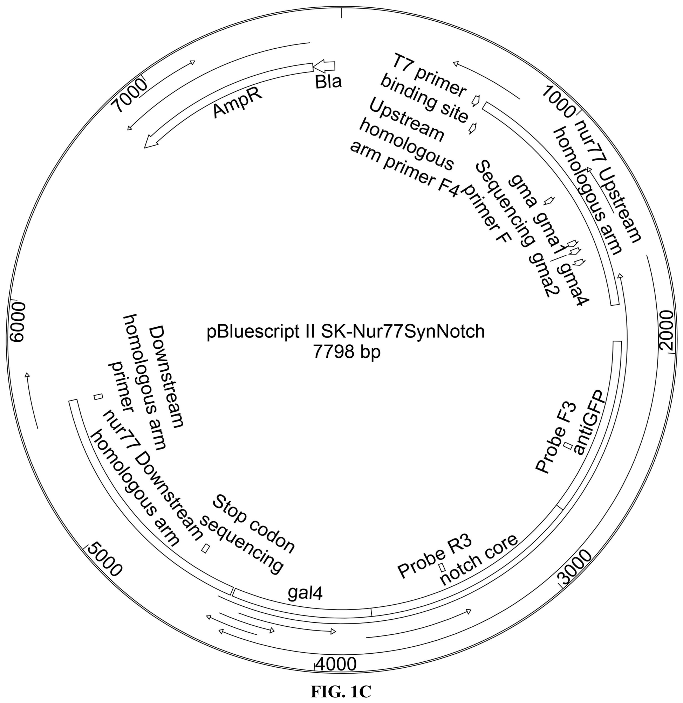

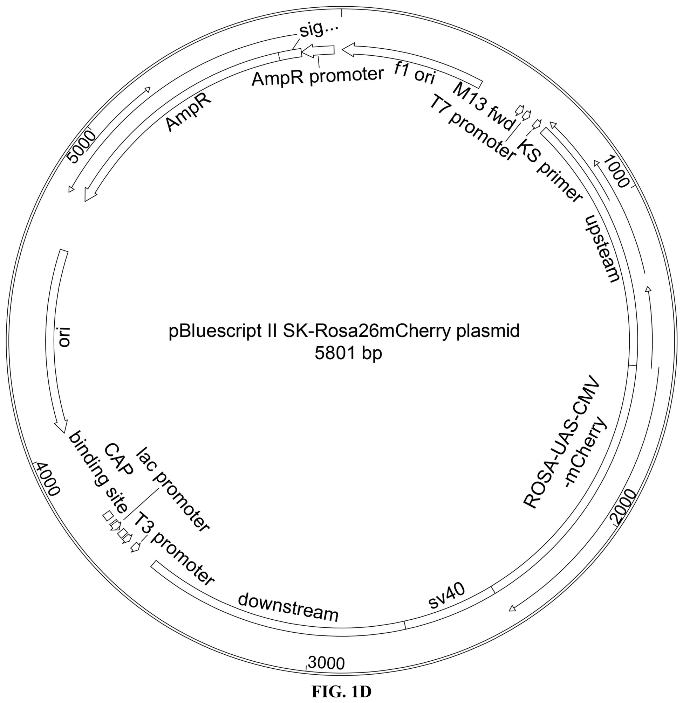

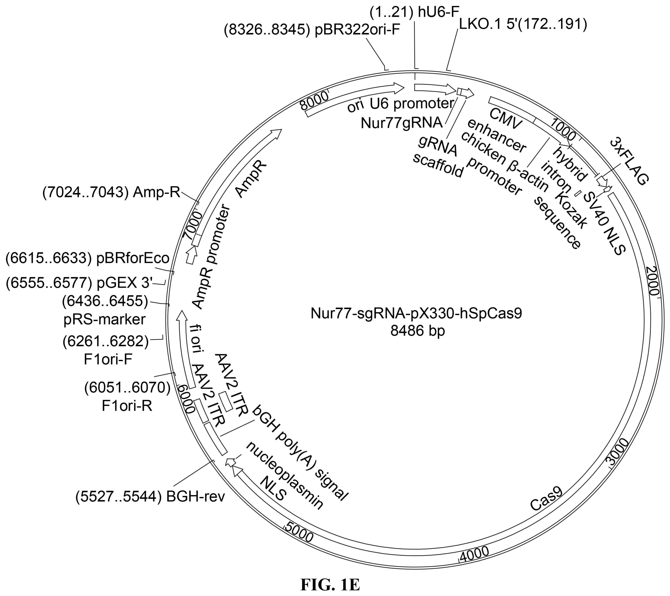

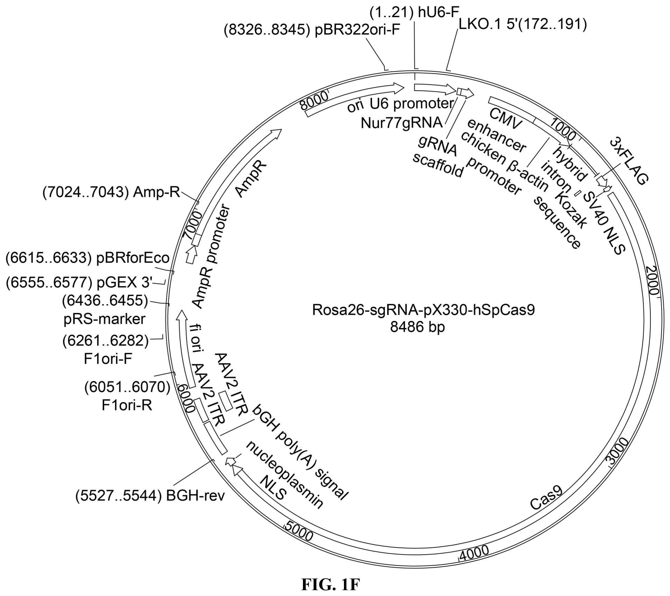

The sequences of the receptor expression system and the activation and screening system were artificially synthesized. Cleavage sites EcoRV and BamHI were added before and after the receptor expression system sequence, respectively, which was then ligated between the upstream and downstream homologous arms flanking the stop codon of the activation gene, followed by ligation into the plasmid pBluescript II SK (the map was shown in FIG. 1c). Cleavage sites NsiI and NheI were added before and after the activation and screening system sequence, respectively, which was then ligated between the upstream and downstream homologous arms of the Rosa26 gene, followed by ligation into the plasmid pBluescript II SK (the map was shown in FIG. 1d). The gRNA was ligated into the DNA cleavage plasmid pX330-U6-Chimeric_BB-CBh-hSpCas9 via the cleavage site BpiI. The maps of the Nur77 gRNA plasmid and the Rosa26 gRNA plasmid are shown in FIG. 1e and FIG. 1f, respectively.

### Example 2

### Construction of mouse model with site-directed knock-in of target gene.

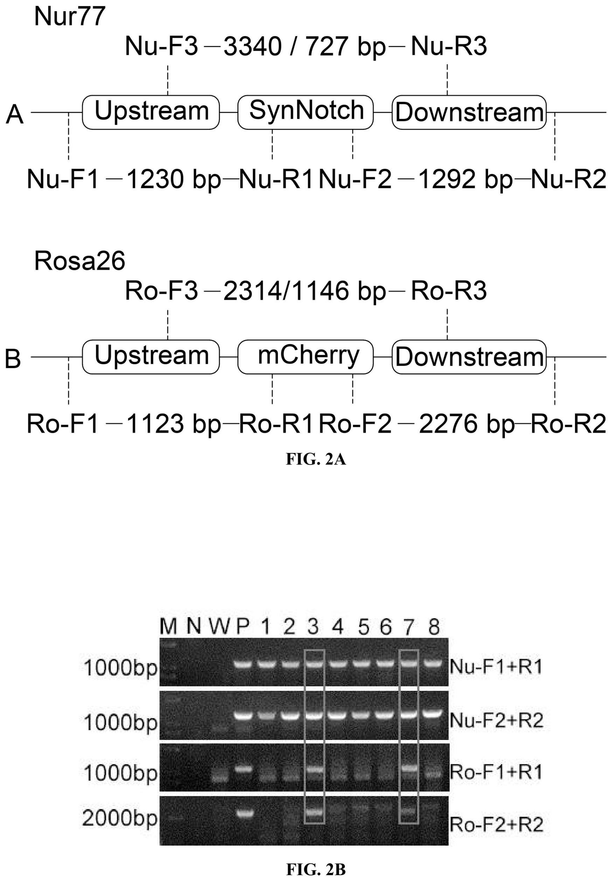

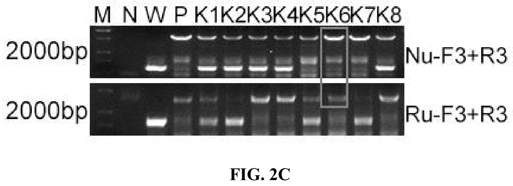

Endotoxin-free large-scale extraction was performed on the target gene plasmids and DNA cleavage plasmids. Separately, the receptor expression system plasmid and the pX330 plasmid, as well as the activation and screening system plasmid and the pX330 plasmid were introduced into fertilized ova of the C57B6L/J mouse via microinjection. The fertilized ova were then transferred into the oviducts of pseudopregnant female mice to obtain F0 generation transgenic mice, followed by genotype identification. For the Nur77 locus: primers Nu-F1 and Nu-R1 were used to detect the insertion of the upstream homologous arm of Nur77, knock-in of 1230 bp; primers Nu-F2 and Nu-R2 were used to detect the insertion of the downstream homologous arm of Nur77, knock-in of 1292 bp; and primers Nu-F3 and Nu-R3 were used for genotyping: 727 bp for the wild-type, 3340 bp + 727 bp for the heterozygote, and 3340 bp for the homozygote. For the Rosa26 locus, primers Ro-F1 and Ro-R1 were used to detect the insertion of the upstream homologous arm of Rosa26, knock-in of 1123 bp; primers Ro-F2 and Ro-R2 were used to detect the insertion of the downstream homologous arm of Rosa26, knock-in of 2276 bp; and primers Ro-F3 and Ro-R3 were used for genotyping: 1146 bp for the wild-type, 2314 bp + 1146 bp for the heterozygote, and 2314 bp for the homozygote (FIG. 2a). The gel electrophoresis image of the genomic PCR detection is shown in FIG. 2b.

The primer sequences were as follows: Nu-F1: 5'-CCGTGGCTTTGGTGATTGGATTGA-3'; Nu-R1: 5'-GGTTCTCCTCCACGTCGCCACAG-3'; Nu-F2: 5'-GCGACGCACTCGATGACTTTGACC-3'; Nu-R2: 5'-CAGAAGGCTAAGAGACAGACCTGAGACCC-3'; Nu-F3: 5'-CTTGAAAGGGTAGGGAGTGAT-3'; Nu-R3: 5'-CTGGAGGGATAAGACAAATAGTG-3'; Ro-F1: 5'-GCTGATTGGCTTCTTTTCCTCCCG-3'; Ro-R1: 5'-CCCTCGATCTCGAACTCGTGGC-3'; Ro-F2: 5'-CCCACAACGAGGACTACAC-3'; Ro-R2: 5'- CCACTGGCTGGCTAAACT-3'; Ro-F3: 5'-GCCTCGTCGTCTGATTGG-3'; Ro-R3: 5'-AGCCTCGATTTGTGGTGTATGT-3'.

### Example 3

### Sorting of tumor antigen-specific B cells.

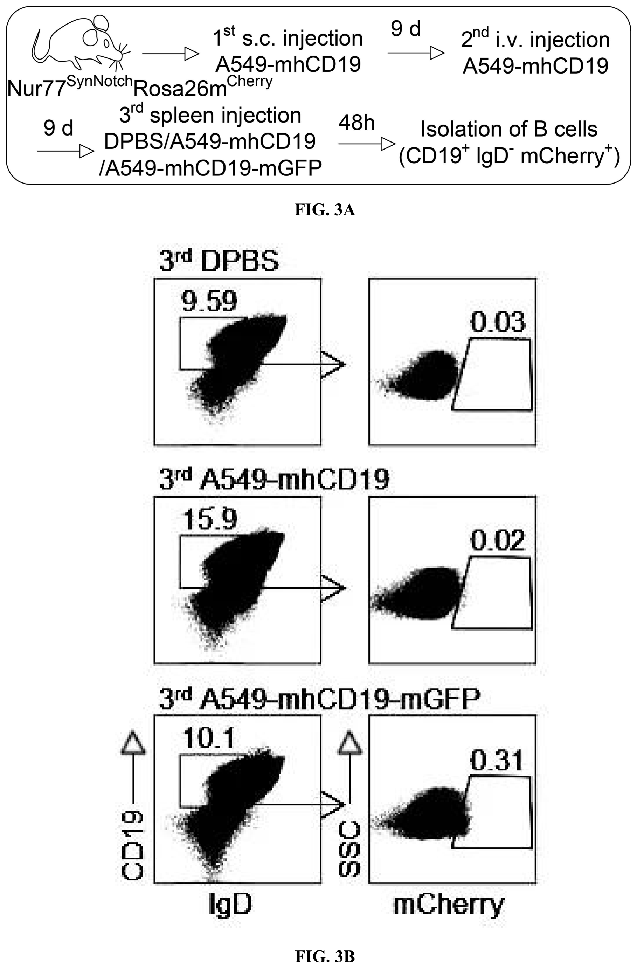

First, the mhCD19 and mGFP proteins were expressed in a membrane-localized manner in the human lung adenocarcinoma cell line A549 using pCDH-CAG as the expression vector. Subsequently, on day 1, 2×10⁶ A549-mhCD19 tumor cells suspended in 200 µl of PBS together with 5 mM CpG ODN 1826 were used to subcutaneously immunize Nur77^{SynNotch}Rosa26^{mCherry} double knock-in mice. On day 9, 2 × 10⁶ A549-mhCD19 tumor cells and 5 mM CpG ODN 1826 were used for intravenous immunization via the tail vein. On day 18, splenic challenge immunization was performed using 2 × 10⁶ A549-mhCD19 or A549-mhCD19-mGFP cells. At 48 h post-challenge immunization, the spleen of the mouse was harvested for the sorting of antigen-specific B cells (FIG. 3a).

Mouse lymphocytes were isolated from splenic lymphocyte separation solution, followed by enrichment of mouse B cells with the Mouse Pan B isolation kit from Miltenyi. After the B cell pellets were resuspended, the final concentration of B cells was adjusted to 1 × 10⁷ cells/mL. The cells were first co-incubated with TruStain FcX^{™} antibody blocking solution at 4°C for 15 min. Then, cell viability staining was performed using the Zombie UV^{™} Fixable Viability Kit, followed by co-incubation at 4°C for 15 min. Then the cells were washed three times with DPBS. Next, an appropriate amount of APC anti-mouse CD19 and FITC anti-mouse IgD flow cytometry direct-labeling antibodies was added for B cell staining, followed by co-incubation at 4°C for 30 min. Then the cells were washed three times with DPBS. Finally, the cells were resuspended and subjected to on-machine sorting of tumor antigen-specific B cells (CD19+IgD-mCherry+) at the single-cell level (FIG. 3b). According to the flow analysis results, the proportion of mCherry-positive cells was 0.02% in Nur77^{SynNotch}Rosa26^{mCherry} double knock-in mice that received challenge immunization with A549-mhCD19 cells, whereas the proportion of mCherry-positive cells was 0.31% in Nur77^{SynNotch}Rosa26^{mCherry} double knock-in mice immunized with A549-mhCD19 cells.

### Example 4

### Acquisition of antibody sequence of tumor antigen-specific B-cells

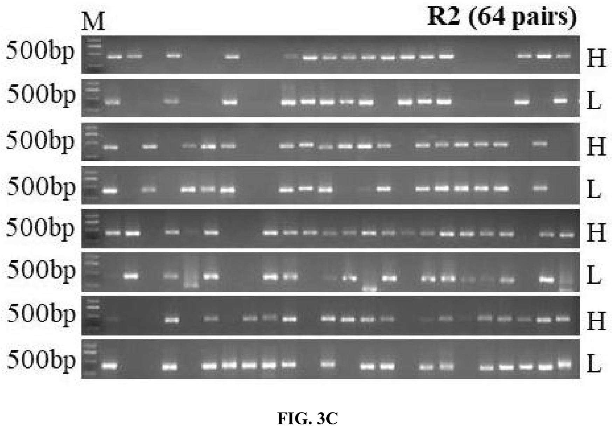

Single tumor antigen-specific B cells (CD19+IgD-mCherry+) were sorted into individual wells of a 96-well plate pre-filled with lysis buffer. Two wells without sorted cells were designated as negative controls. The RNA reverse transcription reaction of single B cells shall be performed in a DNA/RNA-free ultra-clean workbench. The ultra-clean workbench shall be treated with DNA OFFTM and RNaseZap^{™} RNase Decontamination reagent in advance. Sterile and enzyme-free pipette tips shall be used for reverse transcription PCR, followed by amplification of the full-length paired heavy and light chain variable region sequences of a single B cell via the single-cell PCR reaction program. The antibody heavy and light chains were subjected to band size identification using 2% agarose gel electrophoresis, respectively: the target bands of the two-round PCR products were visualized after agarose gel electrophoresis (120 V, 30 min). The size of the heavy chain band was approximately 500 bp, and that of the light chain band was approximately 450 bp (FIG. 3c). The gel blocks containing target bands with correct sizes for both antibody heavy and light chains were purified using a gel extraction kit to obtain the full-length PCR products of paired heavy and light chain variable regions, which were temporarily stored at 4°C for subsequent use. First, the gel-extracted PCR products of heavy or light chain variable region sequences and the recombinant mAb expression vectors for the heavy or light chains were subjected to corresponding enzyme digestion to obtain matched sticky ends: the heavy chain expression vector and the gel-extracted PCR products of heavy chain variable regions were both subjected to enzyme digestion with two restriction endonucleases, AgeI and XhoI; and the light chain expression plasmid and the gel-extracted PCR products of light chain variable regions were both subjected to enzyme digestion with two restriction endonucleases, AgeI and BsiWI. Subsequently, the enzymatically digested heavy chain or light chain expression vector backbones were recovered by gel extraction, and the corresponding digested PCR products of heavy or light chain variable region sequences were purified. The concentrations were determined separately, followed by sticky-end ligation using T4 DNA ligases. The ligation reaction was performed in a 20 µL T4 DNA ligase reaction system at 25°C for 2 h. A 5 µL aliquot of the ligation product was added to 50 µL of competent cells that had been thawed on ice. The mixture was kept on ice for 30 min, heat-shocked in a 42°C water bath for 20 s, and immediately returned to ice for 2 min. Then, 500 µL of low-salt LB medium (without antibiotics) was added, and the cells were activated by shaking at 200 rpm and 37°C for 1 h, and then spread onto low-salt LB plates containing bleomycin for selection. The plates were cultured in a constant-temperature incubator at 37°C for 16 h. Single bacterial colonies were picked and inoculated into 5 mL of low-salt LB liquid medium containing bleomycin, followed by shaking culture at 37°C, 200 rpm for 12 h. The bacterial strains were preserved in glycerol, and plasmids were extracted in small quantities and subjected to sequencing.

### Example 5

### Transient transfection with PEI for recombinant mAb expression in vitro

293T cells were digested and counted, and 5 × 10⁵ cells were seeded in a 10 cm culture dish, followed by culture in a 37°C, CO₂ incubator for 12 h prior to transient transfection with PEI. 10 µg of each of the paired plasmids, namely pFUSE-IL2SS-CHIg-mG1 and pFUSE-IL2SS-CLIg-mG1, was added into 500 µL of opti-MEM medium, and the mixture was mixed thoroughly and kept at room temperature for 1 min. 60 µl of PEI solution (1 µg/µL) was added into 500 µL of opti-MEM medium, and the mixture was mixed thoroughly and kept at room temperature for 1 min. Subsequently, the EP tube containing the plasmids was gently shaken on a vortex shaker, and then the diluted PEI solution was added dropwise at a constant rate. After thorough mixing, the mixture was kept at room temperature for 25 min to form a DNA-PEI complex. Meanwhile, the DMEM growth medium for the 293T cells was discarded and replaced with serum-free medium, after which the DNA-PEI complex formed during the reaction was added dropwise at a constant rate. At 1.5 h post-transfection, a half volume of complete DMEM growth medium (containing 30% serum) was added. At 12 h post-culture, the supernatant of the 293T cell culture medium was discarded, and 10 mL of fresh complete DMEM medium was carefully and slowly added dropwise. After further culture in a 37°C, CO₂ incubator for 36 h, the supernatant of the culture medium containing recombinant mAbs was collected, filtered through a 0.45 µm filter, and transferred to a 30 kDa Amicon Ultra ultrafiltration tube. The tube was centrifuged at 4°C, 4000 rpm for 20 min, and the concentrated mAb supernatant was then collected, mixed thoroughly, aliquoted, and stored at -80°C for subsequent use.

### Example 6

### Screening of recombinant mAbs recognizing target cells

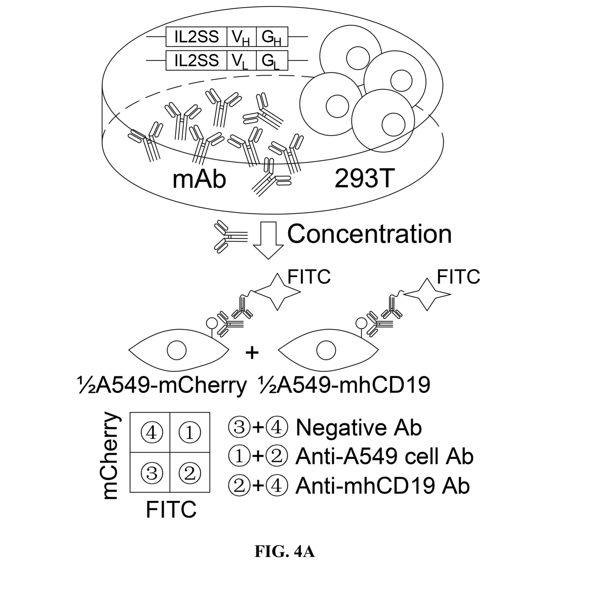

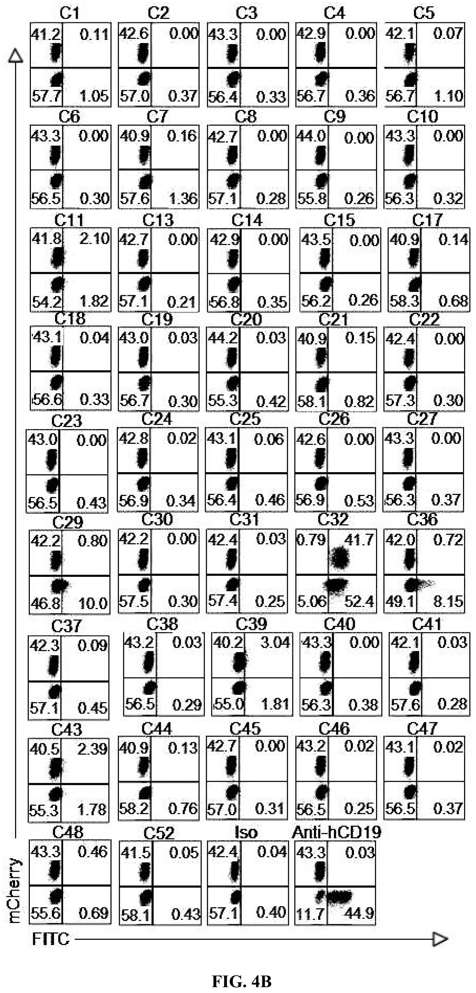

A549-mOVA-mCherry and A549-mhCD19 cells were digested, washed once with DPBS, and then counted. A total of 1 × 10⁶ tumor cells were then collected at a 1 : 1 cell ratio and washed once with DPBS. The cell pellets were resuspended in 100 µL of DPBS, incubated with TruStain FcX^{™} Antibody blocking solution at 4°C for 15 min, followed by the addition of 1 µg of recombinant mAbs and incubation at 4°C for 30 min. After three washes with DPBS, the cell pellets were resuspended in 100 µL of DPBS, the secondary antibody FITC anti-mouse-IgG (1 : 500) was added, and the mixture was incubated at 4°C for 30 min, followed by three washes with DPBS. Finally, the ratios of mCherry and FITC fluorescence were analyzed by FCM to evaluate the capability of the recombinant mAbs to recognize protein antigens on the tumor cell membrane. The specific analysis protocol is shown in FIG. 4a: antibodies corresponding to cell populations distributed in quadrants 3 and 4 are negative antibodies; antibodies corresponding to cell populations distributed in quadrants 1 and 2 are those that recognize A549 cells; and antibodies corresponding to cell populations distributed in quadrants 2 and 4 are those that recognize mCD19. FIG. 4b shows the flow cytometry results for screening recombinant antibodies, in which the recombinant antibody numbered C32 recognizes the A549 cell line.

### Example 7 ADCC

### ADCC assay

(1) Target cells (A549, H23, MCF7, RAG, and CT26) were diluted to 1 × 10⁶ cells/mL. 25 µL of the target cell suspension (equivalent to 2.5 × 10⁴ cells per well) was added to each well of a U-bottom 96-well plate. The antibody (diluted to 20 nM) and the target cells were co-incubated. The following control groups were set up: target cell control, effector cell control, antibody-free control, target cell maximum LDH release control, and Human IgG1 antibody negative control. Three replicate wells were set for each concentration, and incubation was performed at 37°C with 5% CO₂ for 30 min. Peripheral blood mononuclear cells (PBMCs) were collected as effector cells, centrifuged at 1500 rpm at room temperature for 5 min. The cell concentration was adjusted according to an effector-to-target ratio of 20:1. After the antibodies and the target cells were incubated for 30 min, 75 µL of effector cells was added. Centrifugation was performed at 300 g for 5 min, followed by overnight culture in an incubator at 37°C with 5% CO₂.
(2) LDH assay: at 45 min prior to the end of the incubation, target cells in the target cell maximum LDH release control wells were lysed by adding 10 µL of a lysis solution (10×) to determine the maximum LDH release of the target cells. Subsequently, 50 µL of supernatant from all wells was transferred to a flat-bottom 96-well plate. After adding 50 µL of CytoTox96 reagent to each well, the plate was incubated at room temperature in the dark for 30 min. 50 µL of a stop solution was added to each well to terminate the reaction, followed by shaking for 30 s to mix thoroughly. The absorbance value was then measured at 490 nm using a microplate reader. Calculation of cytotoxicity: (Absorbance of antibody well - Absorbance of antibody-free well) / (Absorbance of maximum LDH release control well - Absorbance of target cell control well) × 100.

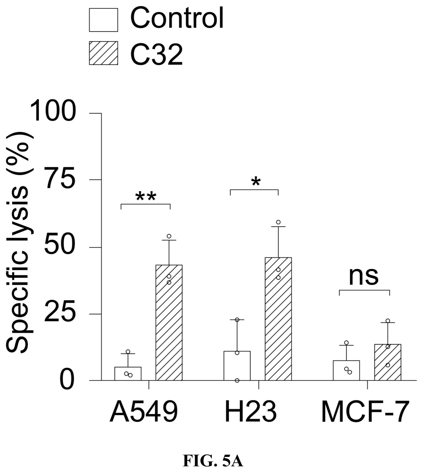

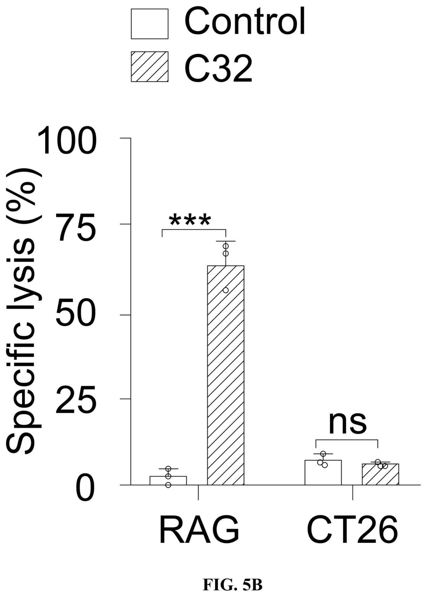

The assay results demonstrate that the C32 antibody can effectively mediate the killing of tumor cells by immune cells (FIG. 5).

Those listed above are merely the optimal particular examples of the present disclosure. Obviously, the present disclosure is not limited to the above examples, and many variations are possible. All variations that can be directly derived or conceived by those skilled in the art from the present disclosure should be considered to be the scope of protection of the present disclosure.

## Claims

1. A method for constructing an animal model, wherein the method comprises: knocking in a synthetic receptor gene into the B cell activation gene Nur77 and knocking in a screening and detection gene into the conserved sequence of gene Rosa26 of genome.

2. The method of claim 1, wherein the synthetic receptor comprises a leader sequence, a tag sequence, an extracellular receptor sequence, a transmembrane region, and an intracellular activation reporter.

3. The method of claim 1, wherein the screening and detection gene comprises a binding domain, a promoter, and a marker gene.

4. The method of claim 1, further comprising: adding cleavage sites before and after synthetic receptor gene SynNotch, and then inserting the synthetic receptor gene SynNotch and the cleavage sites between upstream and downstream homologous arms before stop codon of the gene Nur77; and adding cleavage sites before and after screening and detection gene mCherry, and then inserting the screening and detection gene mCherry, and cleavage sites between upstream and downstream homologous arms of intron of gene Rosa26.

5. The method of any one of claims 1-4, wherein the animal model is a mouse, a rat, a guinea pig, a hamster, a gerbil, a cotton rat, a pig, a cow, sheep, a rabbit, a cat, a dog, a rhesus monkey, an chimpanzee, a baboon, a marmoset, a cynomolgus monkey, or an alpaca, preferably a mouse.

6. The method of any one of claims 1-4, the method further comprising:
1) construction of transgenic vectors: adding cleavage sites before and after SynNotch receptor expression sequence, inserting the SynNotch receptor expression sequence and the cleavage sites between upstream and downstream homologous arms before stop codon of the gene Nur77, and then ligating the gene Nur77, inserted with the SynNotch receptor expression sequence and the cleavage sites, into plasmid pBluescript II SK to obtain a transgenic vector pBluescript II SK-Nur77SynNotch; adding cleavage sites before and after mCherry selection sequence, inserting the mCherry selection sequence and the cleavage sites between the upstream and downstream homologous arms of the intron of the Rosa26 gene, and then ligating the Rosa26 gene, inserted with the mCherry selection sequence and the cleavage sites, into plasmid pBluescript II SK to obtain a transgenic vector pBluescript II SK-Rosa26mCherry; constructing sgRNA-knockout plasmids for both the SynNotch and mCherry sequences, respectively;
2) acquisition of gene-edited animal model: combining the transgenic vector pBluescript II SK-Nur77SynNotch and the sgRNA-knockout plasmid for SynNotch, and combining the pBluescript II SK-Rosa26mCherry plasmid and the sgRNA-knockout plasmid for mCherry to obtain combinations, and introducing the combinations into a fertilized animal ovum via microinjection, respectively to obtain a F0 generation Nur77^{SynNotch}- or Rosa26^{mCherry}-knockin animal; and further carrying out crossing to obtain a homozygous Nur77^{SynNotch}Rosa26^{mCherry} double knock-in animal model.

7. An animal model obtained according to the method of any one of claims 1-6.

8. A method for obtaining a tumor antigen-specific B cell, wherein the method comprises: immunizing the animal model obtained by any one of claims 1-6 or the animal model of claim 7 with a tumor cell, and sorting a tumor antigen-specific CD19+IgD-mCherry+B cell.

9. A tumor antigen-specific B cell obtained according to the method of claim 8.

10. Use of the tumor antigen-specific B cell of claim 9 in screening of a tumor antigen-specific antibody.

11. A transgenic vector combination for use in the preparation of an animal model, comprising:
transgenic vector 1, obtained by a construction method comprising: adding cleavage sites before and after SynNotch receptor expression sequence, inserting the SynNotch receptor expression sequence and the cleavage sites between upstream and downstream homologous arms before stop codon of gene Nur77, and then ligating same into a plasmid to obtain the transgenic vector 1; and
transgenic vector 2, obtained by a construction method comprising: adding cleavage sites before and after mCherry selection sequence, inserting the mCherry selection sequence and the cleavage sites between upstream and downstream homologous arms of intron of gene Rosa26, and then ligating same into a plasmid to obtain the transgenic vector 2.
